# EUROPEAN PATENT SPECIFICATION

(11) **EP 0 874 641 B1**
(45) Date of publication and mention of the grant of the patent: **09.04.2003**
(21) Application number: 96942947.1
(22) Date of filing: 11.12.1996
(51) Int. Cl.: A61K 38/30, A61P 25/00, A61P 25/14, A61P 25/16, A61P 25/28

(54) **IGF-I AND -II FOR THE TREATMENT OF DISEASES OF THE CENTRAL NERVOUS SYSTEM**
IGF-I AND -II ZUR BEHANDLUNG VON KRANKHEITEN IM ZENTRALNERVENSYSTEM
IGF-I ET -II DESTINES AU TRAITEMENT DES MALADIES DU SYSTEME NERVEUX CENTRAL

(30) Priority: 13.12.1995 US 571802
(43) Date of publication of application: 04.11.1998
(73) Proprietor: Aurogen Incorporated, Fort Collins, CO 80522 (US)
(72) Inventor: ISHII, Douglas N., La Porte, CO 80535 (US)
(74) Representative: Wichmann, Hendrik, Dr.
(86) International application number: US9619663
(87) International publication number: WO97021449

(56) References cited:
- WO-A-90/14838
- WO-A-93/02695
- WO-A-93/08828
- WO-A-95/13823
- ENDOCRINOLOGY, vol. 135, no. 5, November 1994, PHILADELPHIA, US, pages 1753-1761, XP000647840 R.R. REINHARDT ETAL.: "INSULIN-LIKE GROWTH FACTORS CROSS THE BLOOD-BRAIN BARRIER." cited in the application

## Description

The invention generally relates to the use of insulin-like growth factor I (IGF-I) or insulin-like growth factor II (IGF-II) for effecting changes in the central nervous system. More particularly, the invention is directed to the manufacture of a medicament for treating disorders or diseases of the brain or spinal cord by parenteral administration of IGF-I or IGF-II.

Many people suffer from disorders and diseases of the brain such as Alzheimer's Disease, Parkinson's Disease, dementia associated with Acquired Immunodeficiency Syndrome (AIDS), Pick's Disease, Huntington's Disease, memory loss due to aging, derangements of the intellect and behavior, neurologic effects of aging, and the like. The treatment of a brain disorder or disease is generally more complicated than treatment of the peripheral nervous system due to the blood-brain barrier which poses an additional obstacle to the delivery of pharmaceutical agents to the brain.

The endothelial cells lining the brain vasculature separate the brain from the blood. This "blood-brain barrier" has been reviewed in Friedemann (1942); Rowland et al. (1991); and Schlosshauer (1993). The blood-brain barrier protects the brain, e.g., from changes in circulating levels of ions, neurotransmitters and growth-altering factors. For example, if high concentrations of certain neurotransmitters were to enter the brain from blood, brain neurons may become inappropriately activated and overexcitation might cause brain disorder or damage.

The brain capillaries which make up the blood-brain barrier are lined with endothelial cells cemented together with very tight junctions, which have few transendothelial channels and allow only scanty pinocytosis. By contrast, the capillaries of the peripheral tissues are lined with endothelial cells which are loosely cemented together with 30-80 Angstrom diameter pores at their junctions, and which have many more transendothelial channels and allow abundant pinocytosis. The tight junctions between the endothelial cells in the blood-brain barrier limit the kinds of molecules that can effectively cross the blood-brain barrier to enter the brain. These molecules include essential molecules needed for brain metabolism and for which there is a specific transport system, such as glucose and amino acids. In addition, small lipophilic molecules can dissolve in the lipoid environment of the endothelial cell plasma membrane and passively diffuse into the brain. By contrast, polar, ionized and large molecules, including proteins, are typically excluded from the brain by the blood-brain barrier.

Similarly, the spinal cord is protected by a blood-spinal cord barrier. For example, spinal cord interneurons have their cell bodies and neuritic processes entirely within the blood-spinal cord barrier. As for the brain. there is a need for a method to effect changes in or treat the mature spinal cord.

Various procedures have been contemplated in efforts to circumvent the blood-brain barrier and effect changes in the brain. For example. in one approach, a small hole is drilled through the skull through which neurotrophic growth factors might be applied to the ventricles of the brain via a catheter, or through which injections or implants can be made. Implanted gel foam, tissues or cells might be used to release such growth factors into the brain. However, such invasive procedures are understandably difficult, risky and require costly surgical procedure. Alternatively, it may be possible to encapsulate neurotrophic proteins within lipid vesicles, and use such vesicles to enhance delivery of factors across the blood-brain barrier.

In another approach, U.S. Patent No. 4,801,575 to Pardridge discloses chimeric peptides wherein a hydrophilic neuropeptide is conjugated via a covalent bond to a transportable peptide for delivering the neuropeptide to the brain. Pardridge discloses such chimeric peptides where the transportable peptide is insulin, transferrin, insulin-like growth factor I (IGF-I), insulin-like growth factor II (IGF-II), basic albumin or prolactin, and where the neuropharmaceutical agent is somatostatin, thyrotropin releasing hormone, vasopressin, alpha interferon or endorphin. However, Pardridge does not employ IGF-I or IGF-II as an agent which itself is effective for treating a brain disorder or disease.

With regard to the spinal cord, previous studies have shown that IGF-I and IGF-II can enhance neurite outgrowth in cultured embryonic rat spinal cord neurons (Ishii et al. (1989)). However, the use of IGF-I and IGF-II to treat neurons within the mature spinal cord, e.g. having a developed blood-spinal cord barrier, particularly for the treatment of disorders and diseases of the mature spinal cord, has not been investigated.

IGF-I and IGF-II are approximately Mᵣ 7500-7700 in size. The neurotrophic properties of the IGFs are discussed in Ishii and Recio-Pinto (1987). IGF receptors are found in brain tissues (Sara et al. (1982); Goodyear et al. (1984)) and are present on neurons and neuroglia cells. IGFs have been shown to prevent the death of cultured embryonic chick sensory and sympathetic neurons and to promote neurite outgrowth (Recio-Pinto et al. (1986)).

Researchers have reported that ¹²⁵I-labeled IGF-I and IGF-II cross the blood-brain barrier and selectively accumulate in specific hypothalamic and anterior thalamic nuclei (Reinhardt and Bondy (1994)). However, those researchers acknowledged that the physiological consequence of IGFs' apparent ability to cross the blood-brain barrier required further study.

WO 93/08828 relates to a method for treating or preventing neuronal damage in the central nervous system comprising parenteral administration outside of the blood-brain barrier or blood-spinal cord barrier to a mammal in need thereof a therapeutically effective amount of a pharmaceutical composition comprising a neurotrophic factor selected from IGF-I and IGF-II and a pharmaceutically acceptable carrier.

The effect of parenterally administered IGF-I or IGF-II on the central nervous system for treatment of brain and spinal cord disorders or diseases as claimed has not yet been determined. Moreover, such determinations are not predictable in view of the blood-brain barrier and blood-spinal cord barrier as obstacles to drug delivery, particularly for large protein molecules such as IGF-I or IGF-II.

Therefore, there is a need in the art of biotechnology and the biopharmaceutical industries for a method of effecting changes to the central nervous system by the administration of large protein molecules across the blood-brain barrier and blood-spinal cord barrier, particularly where the method of treatment involves administration of IGF-I or IGF-II.

The invention relates to the use an IGF-I or an IGF-II for the manufacture of a medicament for treating or preventing neuronal damage in the central nervous system, as claimed. This invention relates to the use of IGFs to ameliorate damage to or treat brain and spinal cord disorders or disease by the use of parenteral administration of IGF-I and/or IGF-II in a pharmaceutical composition, as defined in the claims.

For purposes of this invention, several terms are defined below.

"IGF-I" refers to insulin-like growth factor I, and is also known as IGFI, IGF1, IGF-1 or somatomedin C. For purposes of the invention IGF-I also encompasses homologs of IGF-I from various animal species, whether extracted from tissues or derived as products of recombinant genetic expression vectors, and IGF molecules with substantial sequence homology to human and animal IGF-I that bind to type I IGF receptors. For purposes of the invention, IGF-I does not, however, encompass fusion proteins of IGF-I and a non-IGF peptide, wherein IGF-I functions as a transportable peptide and not as a pharmaceutical agent for treatment of a disorder or disease.

"IGF-II" refers to insulin-like growth factor II, and is also known as IGFII, IGF2 or IGF-2. The invention also encompasses homologs of IGF-II from various animal species, whether extracted from tissues or derived as products of recombinant genetic expression vectors, and IGF molecules with substantial sequence homology to human and animal IGF-II that bind to type I or type II IGF receptors. For purposes of the invention, IGF-II does not, however, encompass fusion proteins of IGF-II and a non-IGF protein, wherein IGF-II functions as a transportable peptide and not as a pharmaceutical agent for treatment of a disorder or disease.

"Brain" is defined as the mature (post-birth) brain within the blood-brain barrier. For purposes of this invention, the brain does not include circumventricular organs such as the pituitary.

"Spinal cord" is defined as the mature (post-birth) spinal cord contained within the blood-spinal cord barrier. For purposes of this invention, the spinal cord does not include neurons, such as motor neurons, whose axons lie in peripheral nerves outside of the blood-spinal cord barrier.

FIG. 1 is a graphical representation of the results of parenteral administration of IGF-I to diabetic and nondiabetic adult rats. The graph shows that administration of IGF-I effectively prevents impairment of IGF-II gene expression in the brain of adult diabetic rats.

FIG. 2A is a graphical representation of the limb withdrawal reflex (lower tracing) and muscle EMG (upper tracing) in a control rat exposed to surgery, but with no harm to the locus ceruleus cells.

FIG. 2B is a graphical representation of the limb withdrawal reflex (lower tracing) and muscle EMG (upper tracing) in the lesioned rat exposed to surgery, with damaged locus ceruleus cells and having a subcutaneously implanted miniosmotic pump releasing vehicle (no drug delivery).

FIG. 2C is a graphical representation of the limb withdrawal reflex (lower tracing) and muscle EMG (upper tracing) in the lesioned rat exposed to surgery, with damaged locus ceruleus cells and implanted subcutaneously with a miniosmotic pump releasing 4.8 µg/day recombinant human IGF-II dissolved in a vehicle.

Procedures to purify and obtain physiologically active IGF-I and IGF-II are known in the art (Zumstein and Humbel (1985); Svoboda and Van Wyk (1985)). IGF-I and IGF-II are commercially available as human recombinant factors, and are sold by Upstate Biotechnology, Inc., Lake Placid, New York; GroPep Ltd., Adelaid, Australia; Austral Biologicals, San Ramon, Calif.; and others.

Pharmaceutical compositions to be employed according to the invention comprise an effective amount of IGF-I or IGF-II. IGF-I or IGF-II is present in the pharmaceutical composition in an amount sufficient to have a therapeutic effect on the condition to be treated. In a further preferred embodiment, IGF-I or IGF-II is present in an amount from 0.1% to 100% of the pharmaceutical composition. For example, the IGF-I or IGF-II may be administered in an amount from 0.1 µg/kg/day up to 4 mg/kg/day. As another example, the IGF-I or IGF-II may be administered in an amount from 400 ng/kg/hour up to 160 µg/kg/hour.

As one of ordinary skill in the art will appreciate, the dosage of pharmaceutical compositions can be adjusted as needed for a particular route of administration, weight of subject, and general condition and disorder or disease. of the patient to be treated whether human or veterinary. Appropriate serum glucose monitoring should be done to prevent hypoglycemia. particularly when the higher end of the IGF dosage range is elected. The half-times of elimination, volumes of distribution, daily production rates, and serum concentrations are established pharmacokinetic parameters for IGF-I and IGF-II in normal humans (Guler et al. (1989); Zapf et al. (1981)).

The pharmaceutical compositions employed according to the invention may further comprise an inorganic or organic, solid or liquid. pharmaceutically acceptable carrier which is preferably suitable for parenteral administration. Compositions may optionally contain adjuncts including preservatives, wetting agents, emulsifiers, solubilizing agents, stabilizing agents, buffers, solvents and salts to maintain tonicity and osmotic pressure. Compositions may be sterilized and exist as solids, particulates or powders, solutions, suspensions or emulsions.

The invention employs parenteral administration. Any means of parenteral IGF administration may be utilized in this invention, including intradermal, subcutaneous, intramuscular, intravenous, intra-arterial or intraperitoneal administration. Other means for parenteral IGF administration might include use of a special infusion or slow release device, release of IGFs from implanted cells or device containing cells, or gene therapy into tissues, all outside the blood-brain barrier or blood-spinal cord barrier with the intent of effecting a change on the central nervous system, particularly to treat a brain or spinal cord disorder or disease.

The following examples are provided to enable those of ordinary skill in the art to make and use the methods of the invention. Efforts have been made to ensure accuracy with respect to numbers used to characterize the measured conditions; however, some experimental errors and deviations may be present.

### EXAMPLE I

### Example of a Pharmaceutical Preparation with IGF-I or IGF-II

A dry ampule (1-60 ml) is partially filled with a sterile solution of IGF-I or IGF-II, and optionally pharmaceutical adjuncts or carriers. and lyophilized. The parenteral solution is prepared by adding an appropriate volume of sterile water, saline or 0.001-0.1 M acetic acid. A pharmaceutical packet may contain a desired number of ampules for a course of treatment, optionally together with instructions for use.

### EXAMPLE II

### Parenteral Administration of IGF-I Having Effect on Impaired IGF-II Gene Expression in Metabolically Disordered Brain

Certain conditions may involve significant damage to the brain with a potential breakdown in the blood-brain barrier, for example, severe concussion injury to the brain, large penetrating wounds or infections causing inflammation to the meninges. The parenteral administration of IGFs under such conditions would not conclusively reveal whether IGFs can generally cross the blood-brain barrier in effective amounts and were therefore avoided in these examples.

This example involves the metabolic disorder of diabetes using the well-studied diabetic rat as a model. The blood-brain barrier is more permeable for certain small ions such as sodium and potassium, but not to other small ions and molecules such as chlorine, calcium or sucrose, in rats where diabetes is experimentally induced with streptozotocin (Knudsen et al. (1986); Jakobsen et al. (1987)). It is expected that, if chlorine, calcium or sucrose permeability is not increased in subjects having a diabetic condition, the permeability of the blood-brain barrier is not expected to be reduced for larger protein molecules, such as IGFs.

Adult rats (12-14 weeks old, about 300 grams in body weight) were treated with streptozotocin to induce insulin-deficient diabetes. One week later, diabetic rats were implanted subcutaneously in the mid-back with miniosmotic pumps releasing either recombinant human IGF-I (4.8 µg per day) or vehicle. After two weeks of continuous parenteral administration of IGF-I, the rat brains were stripped of the meninges and assayed for IGF-II mRNA content. Standard biochemical techniques were used to extract RNA from brain and run Northern and slot blots. A rat cDNA containing the entire coding region of rat IGF-II was used to prepare a single-stranded anti-sense ³²P-labeled hybridization probe, which previously was well characterized. The probe does not cross-hybridize to IGF-I mRNA. Autoradiograms were used to measure IGF-II mRNA content in relative densitometric units (rel. units) per mg wet weight brain tissue. Values are means ± SEM (N = 4 rats per group). The experimental methods are more completely described elsewhere (Soares et al. (1985); Ishii et al. (1994)).

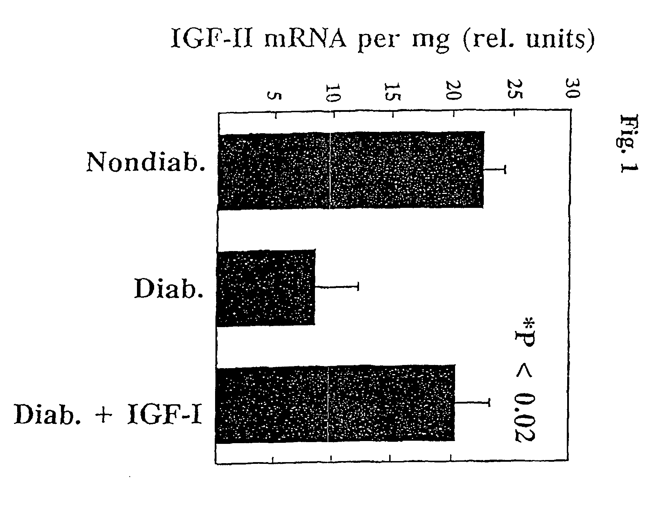

As shown in FIG. 1, IGF-II mRNA content per mg wet weight tissue was significantly reduced in the brains of diabetic adult rats compared to that of nondiabetic rats. This defines a biochemical abnormality in diabetic brain. IGF-II mRNA is produced in various brain regions including hippocampus, thalamus, cerebral cortical layer 5, and choroid plexus (Hynes et al. (1988); Lee et al. (1992)). Neuroglia cells produce IGF-II mRNAs (Rotwein et al. (1988)). The inventor has also found IGF-II mRNA in brain regions such as hippocampus, striatum, midbrain, cerebellum and pons.

As further shown in FIG. 1, the impaired IGF-II gene expression in diabetic brain was ameliorated by parenteral IGF-I. These data show that parenteral administration of IGF-I effects a change in brain, and that it can correct a brain disorder resulting from a disease, in this case diabetes.

This correction of a brain disorder was not due to a significant reduction in hyperglycemia in diabetes, because these same low doses of IGF-I had no effect on hyperglycemia, although they did spare impaired sensory nerve regeneration in diabetic mature rats (Ishii and Lupien (1995)). Other studies have found that the same low doses of IGF-I or IGF-II have no significant effect on either hyperglycemia or weight loss in diabetic adult rats while hyperalgesia (pain) was prevented (Zhuang et al. (1994)). At many-fold higher IGF doses, hyperglycemia or weight loss can be reduced, but this is not required for IGFs to act on the nervous system.

### EXAMPLE III

### Parenteral Administration of IGF-II Having Effect on an Impaired Behavior Resulting from a Brain Lesion

In this model, a chemical lesion produced death of locus ceruleus cells in adult rat brain. Locus ceruleus cells normally project their noradrenergic axons down into the spinal cord to synapse on interneurons which, in turn, modify the activity of motor neurons controlling the hind limb withdrawal reflex. This example tested the capacity of parenterally administered recombinant human IGF-II to preserve this withdrawal reflex. Such preservation would result only if the administered IGF-II were to act on the brain to preserve the noradrenergic axons.

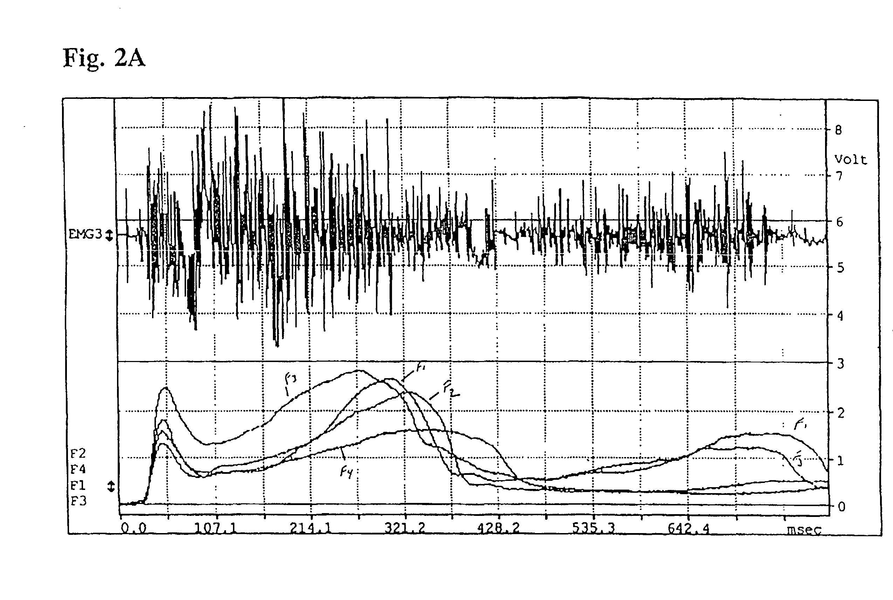

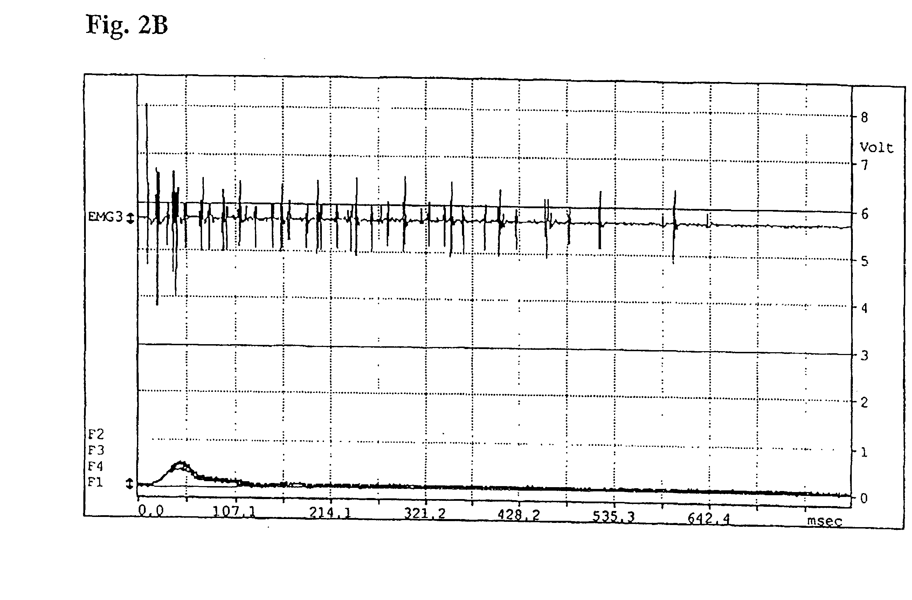

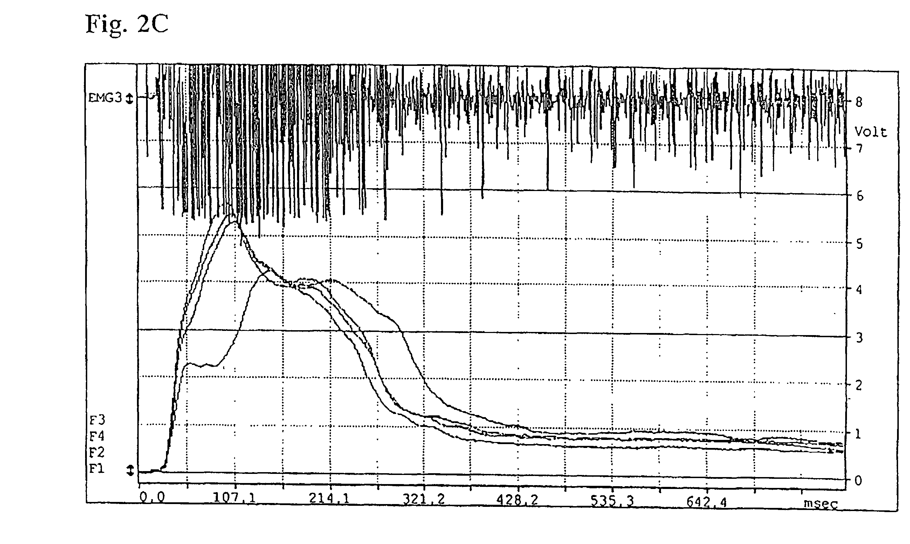

In the experiment shown in FIG. 2, 6-hydroxydopamine (6OHDA) (4 µl of a solution consisting of 12.5 mg 6OHDA per ml and 0.2 mg/ml ascorbic acid in isotonic saline) was injected via a 30 gauge needle into the cisterna magna of adult (12-week-old) Sprague Dawley rats to destroy the noradrenergic locus ceruleus cells in the pons of the brain. Such treatment resulted in the disappearance of the axons from these noradrenergic cells which normally descend down the spinal cord to synapse on interneurons, which, in turn, modulated the activity of motor neurons controlling the limb withdrawal reflex. This reflex was measured by administration of 6.25 mg L-DOPA followed by electrical stimulation which caused release of noradrenaline from the descending noradrenergic fibers of the spinal cord, leading to a large amplitude and long-latency hind limb withdrawal reflex whose force was monitored with a force-displacement transducer. The EMG in muscle was also recorded. Additional experimental details are available from Barnes et al. (1989) and Pulford et al. (1994).

FIG. 2A shows a control rat exposed to surgery and injected with solvent not containing 60HDA. Under this condition there would be no harm to the locus ceruleus cells. The lower tracings show the intact large amplitude and long-latency hind limb withdrawal reflex force. F1-F4 are the force-time tracings which resulted from electrical stimulations of increasing intensity from 1.0-7.5 mA in 2.5 mA increments. The upper tracing shows the associated highly active EMG.

FIG. 2B shows rats lesioned with 6OHDA that were implanted subcutaneously with miniosmotic pumps releasing vehicle alone. The lower tracings show loss of the duration and amplitude of the hind limb reflex force. The upper tracing shows loss of EMG activity.

FIG. 2C shows rats lesioned with 60HDA that were implanted subcutaneously with miniosmotic pumps releasing 4.8 µg/day recombinant human IGF-II. This treatment spared both the hind limb reflex and the EMG activity.

Treatment with 6OHDA is well established by others to result in the loss of noradrenergic brain cells. This was evidenced by the associated loss of the hind limb withdrawal reflex that was measured two weeks later in adult rats (FIG. 2A vs. 2B). A typical example is shown in FIG. 2B. The lower tracing shows the response to the L-DOPA test wherein the large amplitude and long-latency withdrawal reflex present in control unlesioned animals was almost entirely lost in the lesioned rat. The upper tracing shows the loss of EMG activity. These animals were implanted with subcutaneous osmotic minipumps that released vehicle only. In a group of six rats so treated, the average peak force generated was 15.6 ± 3.8 grams (mean, SD) in the hind limb withdrawal test.

By contrast, the hind limb withdrawal reflex was spared in 6OHDA lesioned animals implanted with subcutaneous pumps that released IGF-II (4.8 µg/rat/day) continuously for two weeks. A typical example is shown in FIG. 2C. The lower tracing shows retention of the large amplitude and long-latency withdrawal reflex typical of unlesioned rats. The upper tracing shows retention of EMG activity. In a group of seven rats so treated, the average peak force was 298 ± 38 grams (mean, SD). Therefore, IGF-II treatment caused a significant (P < 0.025) sparing of the reflex vs. vehicle treatment. Thus, the parenteral administration of IGF ameliorated damage, most likely to the noradrenergic locus ceruleus nerve cells in the brain.

The reflex is mediated by noradrenaline, because the response to the L-DOPA test could be blocked by the α-adrenergic blocking agent phentolamine. The procedure producing lesions in the brain does not directly affect the spinal cord motorneurons, because direct electrical stimulation of the motor neuron axons in lesioned animals can cause effective contraction of the hind limb.

During the course of treatments in the above experiments, there were no indications of toxicity due to IGF administration.

The experimental results herein show the new and unexpected observation that parenterally administered IGF-I and IGF-II can effect changes in mature brains of treated rats. Parenteral administration can now be considered a viable route of administration for IGFs for the treatment of brain disorders and diseases, such as Alzheimer's Disease, Parkinson's Disease, dementia associated with Acquired Immunodeficiency Syndrome (AIDS), Pick's Disease, Huntington's Disease, memory losses due to aging, stroke, derangements of the intellect and behavior, neurologic effects of aging, and the like.

The abnormalities in human diabetic brain are well recognized (reviewed by Mooradian et al. (1988); McCall (1991)). These include major depression and cognitive deficits such as loss of memory and complex reasoning skills. Brain atrophy with degeneration of neurons, as well as axonal loss and neurotransmitter imbalances are observed. These are seen in diabetic humans (Reske-Nielsen et al. (1965); Olsson et al. (1968); Soininen et al. (1992)) as well as diabetic rats (Jakobsen et al. (1987); Trulson et al. (1986)). IGF-II is the predominant IGF in brain, and the decline in IGF-II mRNA content in diabetic brain (FIG. 1) suggests that it contributes to the pathogenesis of encephalopathy because IGFs are believed to be necessary for neuron survival, neurite outgrowth and maintenance of synapses. The parenteral administration of IGF-I prevented the decline in IGF-II mRNA within the context of diabetes, and it is expected that such parental administration will be useful to prevent or treat various biochemical disturbances in the diseased, disordered or injured brain.

It is believed that the decline in circulating IGF activity in diabetic patients contributes to abnormalities in diabetic brain. Inasmuch as liver is the primary source of circulating IGFs, other conditions that reduce liver function may contribute to associated brain disorders. For example, in hepatic encephalopathy chronic liver disease or liver failure is associated with altered behavior, impaired cognition, confusion, and coma. The latter carries risk of death or permanent neurological disability. Therefore, the invention further applies to the treatment of the brain in hepatic encephalopathy.

The locus ceruleus cells are located below the cerebellum deep within the pons in the rostral pontine central gray region. Their axons have terminals on intemeurons located entirely within the spinal cord. Because these locus ceruleus noradrenergic neurons and their axons are located entirely within the central nervous system enveloped completely by the blood-brain and blood-spinal cord barriers, it is clear that the parenteral administration of IGF-II effected a change in brain and/or spinal cord (FIG. 2). The parenteral administration of IGF-II was able to prevent the consequences of brain injury. Other studies showed that a single treatment with 2 µg IGF-II mixed together with the 6OHDA (injected through the cisterna magna) did not spare the hind limb withdrawal reflex. Thus, the continuous administration of IGF-II for two weeks was more effective.

IGF-II may have also prevented the secondary consequences of acute injury. For example, following an injury to brain, there can be secondary death of neurons over several days. Because the continuous administration of IGF-II for two weeks was effective whereas a single injection to the brain was not, the continuous administration of IGF appears to provide prophylaxis against secondary. death of neurons or functional damage to neurons.

The most likely explanation is that IGFs crossed the blood-brain barrier in effective amounts.

The invention is expected to be suitable for the treatment of other brain and spinal cord disorders and diseases such as Parkinson's Disease and Alzheimer's Disease. In Parkinson's Disease, there is a loss of dopaminergic neurons in the brain, as well as the noradrenergic neurons in the locus ceruleus. The invention shows that parenteral administration of IGFs can effect the brain system involving these noradrenergic neurons, and such treatment with IGFs is expected to help treat Parkinson's Disease. The dopaminergic and noradrenergic neurons are examples of closely related catecholaminergic neurons, and brain catecholaminergic neurons are lost in Alzheimer's Disease. Neurofibrillary tangles are also observed in the locus ceruleus in Alzheimer's Disease, and such tangles are considered pathogenic. IGFs are expected to help support such locus ceruleus cells, catecholaminergic neurons, and other brain cells weakened in this disease.

The actions of IGFs are not confined to catecholaminergic and locus ceruleus brain cells, and intracranial *IGF* administration can support the survival of a large variety of different brain cells following ischemic injury. Thus, IGF parenteral treatment is expected to be useful in other disorders as well, including lobar atrophy (Pick's disease), Huntington chorea, and various neurodegenerative disorders where many types of neurons are at risk.

Many environmental neurotoxins are well known to cause damage to the brain, for instance, 6OHDA which was used in the above examples as a neurotoxin. Drug abuse with intravenous forms of drugs contaminated with MPTP, which was highly toxic to the dopaminergic neurons of the brain, has also been linked to a Parkinsonian syndrome. IGF treatment can be useful to limit brain damage in patients exposed to such neurotoxins. Environmental neurotoxins may interact with a particular genetic factor to cause the emergence of disorders or diseases such as Alzheimer's Disease.

It is appreciated that aging increases the risk of damage to the brain in many progressive neurodegenerative disorders. For example, individuals with familial forms of Alzheimer's Disease or Huntington's Disease have a genetic disorder, but the disease is not manifest typically until after the fourth or fifth decade in life. "Senile dementia" is another example. Other examples in which age is found to be a factor include but are not limited to cortical-basal ganglionic syndromes, progressive dementia, familial dementia with spastic paraparesis, progressive supranuclear palsy, and Parkinson's Disease. Age is a risk factor in diabetic neuropathy, and this disease includes diabetic encephalopathy.

It is known that circulating IGF levels decline progressively with age (Hall and Sara (1984)). The experiments in this invention suggest that IGF levels may be progressively reduced in the brain as a consequence of this age-dependent decline in circulating IGF levels. Taken together with the knowledge that IGFs are neurotrophic factors and may be maintenance factors for the nervous system, parenteral IGF treatment is particularly helpful to effect changes in the brain, and thereby reduce the risk of damage to the brain in the various disorders in which age is a factor.

There are other brain disorders in which IGFs is expected to be useful. In multiple sclerosis, there is a progressive demyelation of the brain and spinal cord. The etiology includes environmental factors because the prevalence is 50-fold higher in northern climes than in equatorial areas. Age plays a role, and the incidence is low in childhood and high in the third and fourth decade of life. IGFs are known to support myelination, and parenteral forms of IGFs may be useful in this disorder as well as in the diffuse cerebral sclerosis of Schilder in which demyelination is associated with progressive mental deterioration, and acute necrotizing hemorrhagic encephalomyelitis which is a fulminant form of demyelinating disease.

### References

U. Friedemann, "Blood-brain Barrier," *Physiol. Rev.* 22:125-45 (1942).

L.P. Rowland, M.E. Fink and Rubin, "Cerebrospinal Fluid: Blood-brain Barrier, Brain Edema, and Hydrocephalus," *Principles of Neural Science* (E.R. Kandel, J.H. Schwartz and T.M. Jessell eds., 3rd ed., Appleton & Lange 1991).

B. Schlosshauer, "The Blood-brain Barrier: Morphology, Molecules, and Neurothelin, *"BioEssays* 15:341-46 (1993).

D.N. Ishii and E. Recio-Pinto, "Role of Insulin, Insulinlike Growth Factors, and Nerve Growth Factor in Neurite Formation," *Insulin, IGFs, and Their Receptors in the Central Nervous System* 315-48 (M.K. Raizada and M.A. Phillips eds., Plenum Press 1987).

V.R. Sara, K. Hall, H. Von Holtz, R. Humbel, B. Sjogren and L. Wetterberg, "Evidence for the Presence of Specific Receptors for Insulin-like Growth Factors 1 (IGF-1) and 2 (IGF-2) and Insulin Throughout the Adult Human Brain," *Neurosci. Lett.* 34:39-44 (1982).

C.G. Goodyear, L. De Stephano, W.H. Lai, H.J. Guyda and B.I. Posner "Characterization of Insulin-like Growth Factor Receptors in Rat Anterior Pituitary, Hypothalamus, and Brain," *Endocrinology* 114:1187-95 (1984).

E. Recio-Pinto, M.M. Rechler and D.N. Ishii, "Effects of Insulin, Insulinlike Growth Factor-II, and Nerve Growth Factor on Neurite Formation and Survival in Cultured Sympathetic and Sensory Neurons," *J. Neurosci.* 6:1211-19(1986).

R. Reinhardt and C. Bondy, "Insulin Like Growth Factors Cross the Blood-brain Barrier," *Endocrinology* 135:1753-61 (1994).

P.P. Zumstein and R.E. Humbel, "Purification of Human Insulin-like Growth Factors I and II," *Methods in Enzymology* 109:782-87 (1985).

M.E. Svoboda and J.J. Van Wyk, "Purification of Somatomedin-C/Insulinlike Growth Factor I," *Methods in Enzymology* 109:798-816 (1985).

H.P. Guler, J. Zapf, C. Schmid and E.R. Froesch, "Insulin-like Growth Factors I and II in Healthy Man. Estimations of Half-lives and Production Rates," *Acta Endocrinological*(Copenh) 121:753-58 (1989).

J. Zapf, H. Walter, E.R. Froesch, "Radioimmunological Determination of Insulin-like Growth Factors I and II in Normal Subjects and in Patients with Growth Disorders and Extrapancreatic Tumor Hypoglycemia," *J. Clin. Invest.* 68:1321-30(1981).

G.M. Knudsen, J. Jakobsen, M. Juhler and O.B. Paulson, "Decreased Blood-brain Barrier Permeability to Sodium in Early Experimental Diabetes," *Diabetes* 35:1371-73 (1986).

J. Jakobsen, G.M. Knudsen and M. Juhler, "Cation Permeability of the Blood-brain Barrier in Streptozotocin Diabetic Rats," *Diabetologia* 30:409-13 (1987).

M.B. Soares, D.N. Ishii and A. Efstratiadis, "Developmental and Tissue-specific Expression of a Family of Transcripts Related to Rat Insulin-like Growth Factor II mRNA," *Nucl. Acids Res.* 13:1119-34 (1985).

D.N. Ishii, D.M. Guertin and L.R. Whalen, "Reduced Insulin-like Growth Factor-I mRNA Content in Liver, Adrenal Glands and Spinal Cord of Diabetic Rats," *Diabetologia* 37:1073-81 (1994).

M.A. Hynes, P.J. Brooks, J. Van Wyk, and P.K. Lund, "Insulin-like Growth Factor II Messenger Ribonucleic Acids are Synthesized in the Coroid Plexus of the Rat Brain," *Mol. Endocrinol.* 2:45-47 (1988).

W.H. Lee, S. Javedan and C.A. Bondy, "Coordinate Expression of Insulin-like Growth Factor System Components by Neurons and Neuroglia Cells During Retinal and Cerebellar Development," *J. Neurosci,* 12:4737-44 (1992).

P. Rotwein, S.K. Burgess, J.D. Milbrandt, J.E. Krause, "Differential Expression of Insulin-like Growth Factor Genes in Rat Central Nervous System," *Proc. Natl. Acad. Sci.* USA 132:167-73 (1988).

D.N. Ishii and S.B. Lupien, "Insulin-like Growth Factors Protect Against Diabetic Neuropathy: Effects on Sensory Nerve Regeneration in Rats, *J. Neurosci. Res.* 40:138-44(1995).

H.X. Zhuang, S.F. Pu and D.N. Ishii, "Insulin-like Growth Factors (IGFs) Prevent Diabetic Neuropathy (Impaired Nerve Regeneration and Hyperalgesia) in Rats Despite Hyperglycemia," *Soc. Neurosci. Abs.* 20:415 (1994).

C.D. Barnes, S.J. Fung, and O. Pompeiano, "Descending Catecholaminergic Modulation of Spinal Cord Reflexes in Cat and Rat," *Ann. NY Acad. Sci.* 563:45-58(1989).

B.E. Pulford, A.R. Mihajlov, H.O. Nornes and L.R. Whalen, "Effects of Cultured Adrenal Chromaffin Cell Implants on Catecholamine-dependent Hind Limb Reflexes in 6-OHDA Lesioned Rats," *J. Neural. Transplantation & Plasticity* 5:89-102 (1994).

A.D. Mooradian, "Diabetic Complications of the Central Nervous System," *Endocrine Reviews* 9:346-56 (1988).

A.L. McCall, "The Impact of Diabetes on the CNS," *Diabetes* 41:557-70 (1991).

E. Reske-Nielsen, K. Lundbaek, O.U. Rafaelsen, "Pathological Changes in the Central and Peripheral Nervous Systems of Young Long-term Diabetics. I. Diabetic Encephalopathy, *"Diabetologia* 1:233-41 (1965).

Y. Olsson, J. Save-Soderbergh, P. Sourander and L.A. Angervall, "Patho-anatomical Study of the Central and Peripheral Nervous System in Diabetes of Early Onset and Long Duration," *Pathol. Eur.* 3:62-79 (1968).

H. Soininen, M. Puranen, E.L. Helkala, M. Laakson and P. Riekkinen, "Diabetes Mellitus and Brain Atrophy: A Computerized Tomography Study in an Elderly Population," *Neurobiol. Aging* 13:717-21 (1992).

J. Jakobsen, P. Sidenius, H.J.G. Gundersen, and R. Osterby, "Quantitative Changes of Cerebral Neocortical Structures in Insulin-treated Long-term Streptozotocin-diabetic Rats," *Diabetes* 36:597-606 (1987).

M.E. Trulson, J.H. Jacoby and R.G. MacKenzie, "Streptozotocin-induced Diabetes Reduces Brain Serotonin Synthesis in Rats," *J. Neurochem.* 46:1068-72 (1986).

## Claims

1. The use of an IGF-I or an IGF-II *for* the manufacture of a medicament for treating or preventing neuronal damage in the central nervous system, due to AIDS-related dementia, Alzheimer's Disease, Parkinson's Disease, Pick's Disease, Huntington's Disease, hepatic encephalopathy, cortical-basal ganglionic syndromes, progressive dementia, familial dementia with spastic parapavresis, progressive supranuclear palsy, multiple sclerosis, cerebral sclerosis of Schilder or acute necrotizing hemorrhagic encephalomyelitis, wherein the medicament is in a form for parenteral administration of an effective amount of said IGF outside the blood-brain barrier or blood-spinal cord barrier.

2. The use of claim 1 for treating or preventing neuronal damage due to a disorder or disease in the brain.

3. The use of claim 1 or 2, wherein said disorder or disease is Alzheimer's Disease.

4. The use of claim 1 or 2, wherein said disorder or disease is Parkinson's Disease.

5. The use of claim 1 or 2, wherein said disorder or disease is AIDS dementia.

6. The use of claim 1 for treating or preventing neuronal damage due to a disorder or disease in the spinal cord.

7. The use of any of claims 1 to 6, wherein the medicament is in a form for administration of IGF in an amount from 0.1 µg/kg/day up to 4 mg/kg/day.

8. The use of any of claims 1 to 7, wherein the medicament is in a form for administration of IGF in an amount from 400 ng/kg/hour up to 160 µg/kg/hour.

## Patentansprüche

1. Verwendung eines IGF-I oder eines IGF-II für die Herstellung eines Arzneimittels zur Behandlung oder Prävention eines neuronalen Schadens im Zentralnervensystem auf Grund von AIDS-bedingter Demenz, Alzheimerkrankheit, Parkinsonkrankheit, Pick's Krankheit, Huntington's Krankheit, hepatischer Encephalopathie, Syndromen, die cortical-basale Ganglien betreffen, fortschreitende Demenz, familiäre Demenz mit spastischer Paraparese, fortschreitende supranukleare Lähmung, multiple Sklerose, cerebrale Sklerose (Schilder) oder akute nekrotisierende, hämoragische Enzephalomyelitis, wobei das Arzneimittel in einer Form zur parenteralen Verabreichung einer wirksamen Menge des genannten IGFs außerhalb der Blut-Hirn-Schranke oder Blut-Rückenmarks-Schranke ist.

2. Verwendung nach Anspruch 1 zur Behandlung oder Prävention von neuronalen Schäden auf Grund einer Störung oder Krankheit des Gehirns.

3. Verwendung nach Anspruch 1 oder 2, wobei besagte Störung oder Krankheit die Alzheimerkrankheit ist.

4. Verwendung nach Anspruch 1 oder 2, wobei besagte Störung oder Krankheit die Parkinsonkrankheit ist.

5. Verwendung nach Anspruch 1 oder 2, wobei besagte Störung oder Krankheit die AIDS-Demenz ist.

6. Verwendung nach Anspruch 1 zur Behandlung oder Prävention von neuronalen Schäden auf Grund einer Störung oder Krankheit des Rückenmarks.

7. Verwendung nach irgendeinem der Ansprüche 1 bis 6, wobei das Arzneimittel zur Verabreichung von IGF in einer Menge von 0,1 µg/kg/Tag bis zu 4 µg/kg/Tag formuliert ist.

8. Verwendung nach irgendeinem der Ansprüche 1 bis 7, wobei das Arzneimittel zur Verabreichung von IGF in einer Menge von 400 ng/kg/Stunde bis zu 160 µg/kg/Stunde formuliert ist.

## Revendications

1. L'utilisation d'un facteur IGF-I ou IGF-II pour la fabrication d'un médicament pour le traitement ou la prévention des dommages neuronaux dans le système nerveux central dus à une démence liée au sida, à la maladie d'Alzheimer, à la maladie de Parkinson, à la maladie de Pick, à la maladie de Huntington, à une encéphalopathie hépatique, à des syndromes ganglionnaires corticaux-basaux, à une démence progressive, à une démence familiale avec paraparèse, à une paralysie supranucléaire progressive, à une sclérose multiple, à une sclérose cérébrale de Schilder ou à une encéphalomyélite aiguë nécrosante, où le médicament est sous une forme permettant l'administration d'une quantité efficace de cet IGF hors de la barrière sang-cerveau ou de la barrière sang-moelle épinière.

2. L'utilisation de la revendication 1 pour le traitement ou la prévention des dommages neuronaux dus à un trouble ou une maladie du cerveau.

3. L'utilisation de la revendication 1 ou 2, où ledit trouble ou maladie est la maladie d'Alzheimer.

4. L'utilisation de la revendication 1 ou 2, où ledit trouble ou maladie est la maladie de Parkinson.

5. L'utilisation de la revendication 1 ou 2, où ledit trouble ou maladie est la démence du sida.

6. L'utilisation de la revendication 1 pour le traitement ou la prévention des dommages neuronaux dus à un trouble ou une maladie de la moelle épinière.

7. L'utilisation de l'une des revendications 1 à 6, où le médicament est sous une forme permettant l'administration de l'IGF en une quantité allant de 0,1 µg/kg/jour à 4 mg/kg/jour.

8. L'utilisation de l'une des revendications 1 à 7, où le médicament est sous une forme permettant l'administration de l'IGF en une quantité allant de 400 ng/kg/heure à 160 µg/kg/heure.
